(19) Europäisches Patentamt
European Patent Office
Office européen des brevets

(11) Publication number: 0 289 345
A1

# (12) EUROPEAN PATENT APPLICATION

(21) Application number: 88303922.4

(22) Date of filing: 29.04.88

(51) Int. Cl.[4]: C 12 M 1/40
G 01 N 33/48

(30) Priority: 01.05.87 GB 8710472

(43) Date of publication of application:
02.11.88 Bulletin 88/44

(84) Designated Contracting States:
BE DE ES FR GB GR IT LU NL SE

(71) Applicant: CAMBRIDGE LIFE SCIENCES PLC
Cambridge Science Park Milton Road
Cambridge CB4 4BH (GB)

(72) Inventor: Benetto, Hugh Peter
64 Woodheyes Road
London, NW10 (GB)

Delaney, Gerard Michael
96 St. Stephen's Avenue
London, W12 (GB)

Mason, Jeremy Richard
45 Friar's Place Lane
London, W3 (GB)

Thurston, Christopher Frank
26 Ranelagh Road
London, W5 (GB)

Stirling, John Laing
78 Twyford Avenue
London, W3 (GB)

DeKeyzer, David Robert
19 St. John'sClose
Needingworth Cambridgeshire PE17 3PP (GB)

Mullen, William Henry
61 Matin Close Soham
Ely Cambridgeshire CB7 5EJ (GB)

(74) Representative: Lambert, Hugh Richmond et al
D. YOUNG & CO. 10 Staple Inn
London, WC1V 7RD (GB)

## (54) Amperometric method for the quantitative determination of 1,4-dihydronicotinamide adenine in solution.

(57) A method is disclosed for the quantitative determination of 1,4-dihydronicotinamide adenine dinucleotide (NADH) in solution. The method comprises contacting the NADH-containing solution with an activated carbon electrode, maintaining the carbon electrode at a controlled, fixed potential effective to cause oxidation of NADH at the electrode surface, and measuring the current output from the carbon electrode, wherein there is used an activated carbon electrode comprising a porous, heterogeneous, resin-bonded layer of activated carbon or graphite particles bonded together with a natural or synthetic resin binder, preferably polytetrafluoroethylene.

EP 0 289 345 A1

## Description

# AMPEROMETRIC METHOD FOR THE QUANTITATIVE DETERMINATION OF 1,4-DIHYDRONICOTINAMIDE ADENINE DINUCLEOTIDE (NADH) IN SOLUTION

This invention relates to a method for the quantitative determination of 1,4-dihydronicotinamide adenine dinucleotide (NADH) in solution.

NADH and its oxidised counterpart NAD are cofactors in numerous enzyme catalysed redox reactions. In some, an enzyme substrate is oxidised in the presence of cofactor NAD and a suitable oxidase or dehydrogenase to yield NADH in solution; in others an enzyme substrate is reduced in the presence of cofactor NADH to yield NAD in solution. In many cases, determination of the NADH concentration can be used as an indicator of substrate concentration, or as a means of following the course of an enzyme reaction involving NADH (or NAD).

It is known that NADH concentration in solution can be measured colorimetrically, but colorimetric methods on the whole are disadvantageous. Much more advantageous are electrochemical methods, but attempts to determine NADH electrochemically have so far not met with any very great degree of success. It is known, for example, that NADH concentration can be determined by an amperometric assay in which NADH is oxidised at an electrode at a fixed, controlled, potential, the current passing under suitable conditions being proportional to NADH concentration. Unfortunately the electrochemical oxidation of NADH requires a high overpotential, and the NADH is generally not oxidised cleanly at the electrode surface; for example, in many cases the surface of the electrode is quickly fouled by formation of a surface film which affects the size and speed of the electrochemical response: I. Moiroux and P.J. Elving, J. Amer. Chem. Soc. (1980) 102, 6533-6538, and D.G. Johnson, M.D. Ryan and G.S. Wilson, Analyt. Chem.(1986) 58, 42R.

There have been many attempts to avoid these problems. For example, it has been proposed to use modified electrodes coated with a layer of conducting organic salts: J.J. Kulys, Biosensors (1986) 2, 3-13. Alternatively it has been proposed to use an absorbed redox mediator such as Meldola's blue to couple the oxidation reaction more effectively to the electrode and/or to lower the oxidation potential: L. Gorton et al., J. Electroanalyt. Chem. (1984), 161, 103-20. In another proposal redox mediators have been used in free solution. For example, methoxy phenazine methosulphate has been used with a modified pyrolitic graphite electrode: Y. Kimura and K. Nihi, Analytical Sciences (1985), 1, 271-4. Other experiments have been carried out with platinum, graphite and glassy carbon electrodes, but as yet no electrochemical method for the determination of NADH has been developed which is both rapid and reproducible.

In accordance with the present invention it has been discovered that NADH can be oxidised cleanly, with good amperometic response, both in buffer solutions containing NADH alone, and in solutions containing enzyme, enzyme substrate and NADH, using an activated carbon electrode comprising a heterogeneous resin bonded layer of activated carbon or graphite particles bonded with a natural or synthetic resin, preferably a synthetic hydrophobic resin such as a fluorocarbon resin, and most preferably polytetrafluoroethylene. The resin-bonded layer of activated carbon or graphite particles may be self-supporting, but will usually be supported by a support member, preferably an electrically conductive support member, and preferably a layer of electrically conductive carbon paper to which the resin-bonded activated carbon or graphite particles are bonded as a surface layer, or impregnated into a carbon fibre web, preferably an electrically conductive web.

The preferred enzyme electrode substrates used in accordance with this invention are, in fact available from the Prototech Company of Newton Highlands, Massachussets. In broad detail, these comprise a powdered activated carbon or graphite (particle size 50 to 300 Angstroms: 5 to 30 nm) moulded onto an electrically conductive supporting structure, e.g. electrically conductive carbon paper, using a synthetic hydrophobic resin binder, preferably a fluorinated hydrocarbon resin, and especially polytetrafluoroethylene.

In an alternative, the activated carbon or graphite particles may be impregnated into a preferred porous carbon cloth and bonded therein using the fluorocarbon resin, preferably polytetrafluoroethylene. It is to be understood, however, that the present invention is not limited to the use of Prototech materials, but embraces other similar substrate materials comprising a porous resin-bonded layer of activated carbon or graphite particles and a natural or synthetic, preferably hydrophobic resin binder.

Herein, the term "activated carbon" or "activated graphite" refers to highly porous, high surface area carbon and graphite materials having a surface area in excess of about 30 $m^2/g$, preferably at least about 50 $m^2/g$, and most preferably, at least about 200 $m^2/g$, e.g. in the range 200 to 1800 $m^2/g$. Such high surface materials can be obtained by the heat treatment of carbon or graphite in the presence of steam or $CO_2$, such treatment being effective to increase substantially the available surface area of the carbon or graphite particles usually to values in the range 200 to 1800 $m^2/g$. The activated carbons used herein may have a particle size ranging from as little as 3 nm up to as much as 0.5 mm, i.e. ranging from an extremely fine particle size up to relatively coarse particles. Preferably, however, the carbon or graphite powder has a particle size in the range 3 to 500 nm, most preferably from 5 to 100 nm.

Whilst the preferred resin binders used to bind the activated carbon or graphite particles are synthetic hydrophobic resins, e.g. fluorocarbon resins, particularly polytetrafluoroethylene, other suitable resin binders include polyethylmethacrylate, polyvinyl acetate, polyvinyl chloride, polycarbonates, poly(4-methylpentene-1) polyisoprene, polychloroprene, poly(1,3-butadiene), silicone rubber and gelatin.

The proportion of binder to activated carbon or graphite, on a weight basis, may range from 10 to 75%

binder and 90 to 25% powdered activated carbon or graphite, preferably 20 to 50% binder and correspondingly 80 to 50% activated carbon or graphite.

In comparison to the known glassy carbon or graphite electrodes previously proposed for the quantitative determination of NADH, and which require relatively high operating potentials, i.e. of about 750 mV with reference to the standard Ag/AgCl reference electrode, the present electrodes are operable at much lower potentials, i.e. in the range 0 to 200 mV, against an Ag/AgCl reference electrode, or even lower, i.e. at negative potentials. The ability to operate at such low potentials is of considerable benefit in reducing the levels of background current and in enhancing the stability, reproducibility and sensitivity of the present system. The electrodes are further characterised by their unsensitivity to potentially interfering species, such as uric acid, often present in biological or clinical samples.

Instead of moulding the resin/activated carbon powder directly onto the surface of a suitable support, e.g. directly onto the surface of electrically conductive carbon paper, the mixture of binder and activated carbon powder may be suspended in a suitable inert medium, and applied to the surface of the substrate by a screen printing technique, thereby providing a thin film of resin-bonded activated carbon particles on the surface of the substrate.

As well as the direct quantitative measurement of NADH in solution, the electrodes and process of the present invention may be used in the quantitative determination of NADH generated or consumed in situ for example by the enzymatic reaction between an enzyme and its substrate utilizing NAD (or NADH) as a cofactor. Such reactions include for example the conversion of pyruvate to lactate by lactate dehydrogenase, i.e. the reaction

$$\text{pyruvate} + \text{NADH} \xrightarrow{\text{lactate dehydrogenase}} \text{lactate} + \text{NAD}$$

which reaction may be monitored by the decrease in NADH concentration; and the oxidation of glucose to gluconolactone by the reaction

$$\beta\text{-D-glucose} + \text{NAD} \xrightarrow[\text{dehydrogenase}]{\text{glucose}} \text{D-gluconolactone} + \text{NADH}$$

which can be monitored by the increase in NADH concentration as the reaction proceeds. To this end the activated carbon electrodes used in accordance with this invention may have an enzyme such as lactate dehydrogenase or glucose dehydrogenase incorporated into or immobilised onto the resin bonded carbon layer by any of the enzyme immobilization techniques known in the art and taught for example in EP-A-0 247 850.

In a further modification of this concept the present invention also envisages a one off, disposable enzyme electrode and method in which the enzyme electrode itself comprises not only the immobilised enzyme, but also the appropriate cofactor for that enzyme, either NAD or NADH as the case may be, the enzyme electrode thus having the capability of responding amperometrically to the activity of the enzyme, as determined by the change in NADH concentration, when in contact with a sample, e.g. a clinical or biological sample, containing the relevant substrate for that enzyme, irrespective of whether that sample contains the necessary cofactor, since that is supplied by the electrode itself. The NAD or NADH cofactor may be incorporated into the electrode in any suitable manner such as impregnation with a suitable solution of either NAD or NADH and drying.

As is also known in the art, the surface of the electrode material may or may not be protected by a porous membrane, such as a polycarbonate film having a pore size of for example about 0.03 µm. Other suitable membrane materials may also be used.

The invention is further described with reference to the accompanying drawings, in which:

Figure 1 is a diagrammatic section through the electrode assembly of an electrochemical cell used to test the NADH response of the activated carbon electrodes in accordance with this invention; and

Figures 2, 3 and 4 show the electrode response to various concentrations of NADH using different activated carbon paper electrodes according to this invention.

Referring to Figure 1, the electrode assembly of the electrochemical cell used to test the response of the activated carbon electrodes to NADH comprises a platinum contact button B surrounded by the reference/counter electrode C, being separated from it by an insulating sleeve G. A porous polycarbon membrane (0,03 µm) mounted on an "O" ring D is used to hold the test disc E (the carbon paper electrode material) onto the platinum contact. An open sample chamber F allows samples to be placed dropwise onto the membrane. The electrode cell is polarised at various potentials relative to the Ag/AgCl reference electrode and the current monitored via a potentiostat A.

The results obtained will be discussed in more detail below.

EXAMPLE

Using an electrochemical cell as described above, and an activated carbon paper electrode material supplied by the Prototech Company cut into 1.5 mm discs and positioned on the platinum contact B, the current output was measured at various NADH concentrations with the electrode poised at 0 and 200 mV. The activated carbon paper electrode material comprises a porous layer of activated carbon powder (50% by weight) (Vulcan XC72, nominal particle size 30 nm, surface area approx. 230$m^2$/g) and polytetrafluoroethylene binder (50% by weight) bonded to the surface of an electrically conductive carbon backing paper (Toray backing paper). In an initial run to obtain a stable background current, the electrode material is exposed to a buffer containing 16 mmol/L $NaH_2PO_4$, 53 mmol/L $NaH_2PO_4$, 52 mmol/L NaCl, 1.5 mmol/L ethylenediaminetetraacetic acid, pH 7.4. Once a stable background current is obtained, the buffer was wiped off the surface of the membrane, and replaced by samples of NADH in the same buffer. Peak current outputs were recorded and are presented in Figure 2, which shows the output current density in $\mu$A/$cm^2$ plotted against the NADH concentrations in the sample.

Other similar resin-bonded, activated carbon electrodes have similarly been tested for their response to NADH in solution and the results are as follows. In this case a three electrode cell configuration was used as shown in Figure 1 of EP-A-0 247 850, and the activated carbon paper electrode was used as such, i.e. without any protective membrane. Measurements were made at a concentration of 1.0 mmol/L NADH at 200 mV vs. Ag/Ag/Cl.

| Electrode No. | Activated Carbon Type | Surface Area ($m^2$/g) | Polytetra-fluoroethylene binder, wt% | Current Output (uA/$cm^2$) |
|---|---|---|---|---|
| 1 | Vulcan XC-72 | 230 | 50 | 210 |
| 2 | Vulcan XC-72 | 230 | 30 | 175 |
| 3 | Vulcan XC-72 | 230 | 70 | 310 |
| 4 | Vulcan XC-72* | 81 | 35 | 150 |
| 5 | Black Pearl | 1800 | 50 | 400 |
| 6 | Black Pearl* | 230 | 35 | 200 |
| 7 | Shawinigan Acetylene Black (SAB) | - | 35 | 170 |
| 8 | SAB* | 50 | 35 | 240 |
| 9 | Ketjen Black | 1000 | 50 | 310 |

* After heat treatment at 2700°C

The above identified carbon products are supplied by:

Cabot: Vulcan XC-72 and Black Pearl

Texas Gulf: Shawinigan Acetylene Black SAB

Further experiments were also carried out using various concentrations of NADH using a resin-bonded, activated carbon electrode (Black Pearl, heat treated at 2700°C; surface area 230 $m^2$/g; PTFE binder 35%). The substantially linear response of the electrode to NADH concentration is shown in Figure 3.

To illustrate the screen printing technique, for the manufacture of the activated carbon electrodes, an aqueous paste was prepared by mixing 200 g of activated carbon powder (Vulcan XC-72) into an aqueous gelatin solution (20% w/v) at 37°C. The paste was then spread as a thin film on a piece of electrically conductive carbon paper (Toray backing paper) and left to dry. When dry, the activated carbon electrode material was cut into 1.5 mm discs and tested for its response to NADH in the two electrode cell configuration described above, and with the electrode poised respectively at 0 mV and 200 mV. The substantially linear response of the electrode to NADH concentration is shown in Figure 4.

The above Example demonstrates the use of the electrode material to produce a rapid and reproducible oxidation of NADH. These responses are in marked contrast to those given by most other electrode materials, as demonstrated in comparable experiments using platinum, glassy carbon, or graphite electrode materials, which (with rare exceptions) are generally sluggish, relatively insensitive, and of much poorer reproducibility. The effectiveness of the activated carbon electrodes we have used appears to be a result of their particular

heterogeneous highly porous structure and its compatibility with biological molecules such as NADH and enzymes. The oxidation of NADH also proceeds efficiently in the presence of enzymes and substrates, and can be as a basis for rapid NADH-coupled enzymatic assays.

Although the invention has been described herein solely with reference to the determination of NADH in solution, phosphorylated 1,4-dihydronicotinamide adenine dinucelotide (NADPH), i.e. phosphorylated NADH, may be determined by exactly the same technique. Moreover, since NADPH (or NADP) is a cofactor in a selected group of enzyme catalysed reactions, rather than NADH (or NAD), the technique of this invention enables such reactions to be monitored in exactly the same way, i.e. by amperometrically determining either the consumption or the production of NADPH in solution. Thus all reference herein to NADH, or NAD, are to be taken to include the phosphorylated derivative unless the context requires otherwise.

**Claims**

1. A method for the quantitative determination of 1,4-dihydronicotinamide adenine dinucleotide (NADH) in solution which comprises contacting the NADH-containing solution with an activated carbon electrode, maintaining the carbon electrode at a controlled, fixed potential effective to cause oxidation of NADH at the electrode surface, and measuring the current output from the carbon electrode, wherein there is used an activated carbon electrode comprising a porous, heterogeneous, resin-bonded layer of activated carbon or graphite particles bonded together with a natural or synthetic resin binder.

2. A method according to claim 1, wherein said activated carbon or graphite particles are bonded together with a fluorocarbon resin.

3. A method according to claim 2, wherein said fluorocarbon resin is polytetrafluoroethylene.

4. A method according to any one of claims 1 to 3, wherein the resin-bonded activated carbon or graphite particles are formed as a resin-bonded porous surface layer on an underlying support member.

5. A method according to claim 4, wherein said support member is electrically conductive.

6. A method according to claim 5, wherein said electrically conductive support member is a layer of electrically conductive carbon paper to which said activated carbon or graphite are bonded as a surface layer.

7. A method according to any one of claims 1 to 3, wherein the resin-bonded activated carbon or graphite particles are impregnated into and supported by a carbon fibre web.

8. A method according to any one of claims 1 to 7, wherein said activated carbon or graphite particles have a particle size in the range 5 to 100 nm.

9. A method according to any one of claims 1 to 7, as applied to monitoring the production or consumption of NADH in an enzymatic reaction between an enzyme and its substrate and involving either NADH or NAD as a cofactor.

10. A method according to claim 9, wherein there is used an activated carbon electrode having said enzyme incorporated or immobilised thereon or therein, and in which the electrode is used to monitor the activity of the enzyme via the change of NADH concentration when the electrode is in contact with a sample containing the enzyme substrate and in the presence of either NAD or NADH as a cofactor involved in the reaction between the enzyme and its substrate.

11. A method according to claim 10, wherein there is used an activated carbon electrode having said enzyme incorporated or immobilised thereon or therein in conjunction with the NADH or NAD cofactor, as appropriate, the NADH or NAD cofactor thus being introduced into the sample via the electrode.

12. A one off, disposable enzyme electrode for use in the method of claim 11, comprising an activated carbon electrode consisting of or comprising a porous, resin-bonded heterogeneous layer of activated carbon or graphite particles bonded together with a synthetic hydrophobic resin binder, said resin-bonded layer of activated carbon or graphite particles having an enzyme immobilised therein or thereon in conjunction with NAD or NADH as a cofactor for said enzyme, said electrode responding amperometrically to the activity of said enzyme, when in the presence of its substrate, and said NAD or NADH as the case may be.

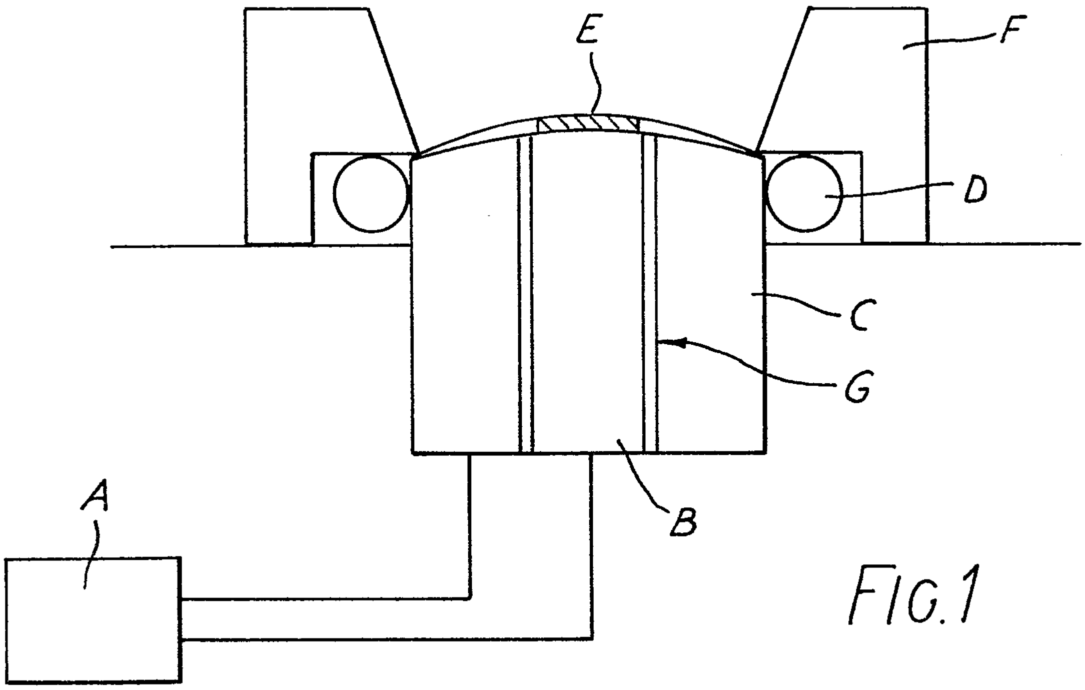
E
F
D
C
G
A
B

FIG.1

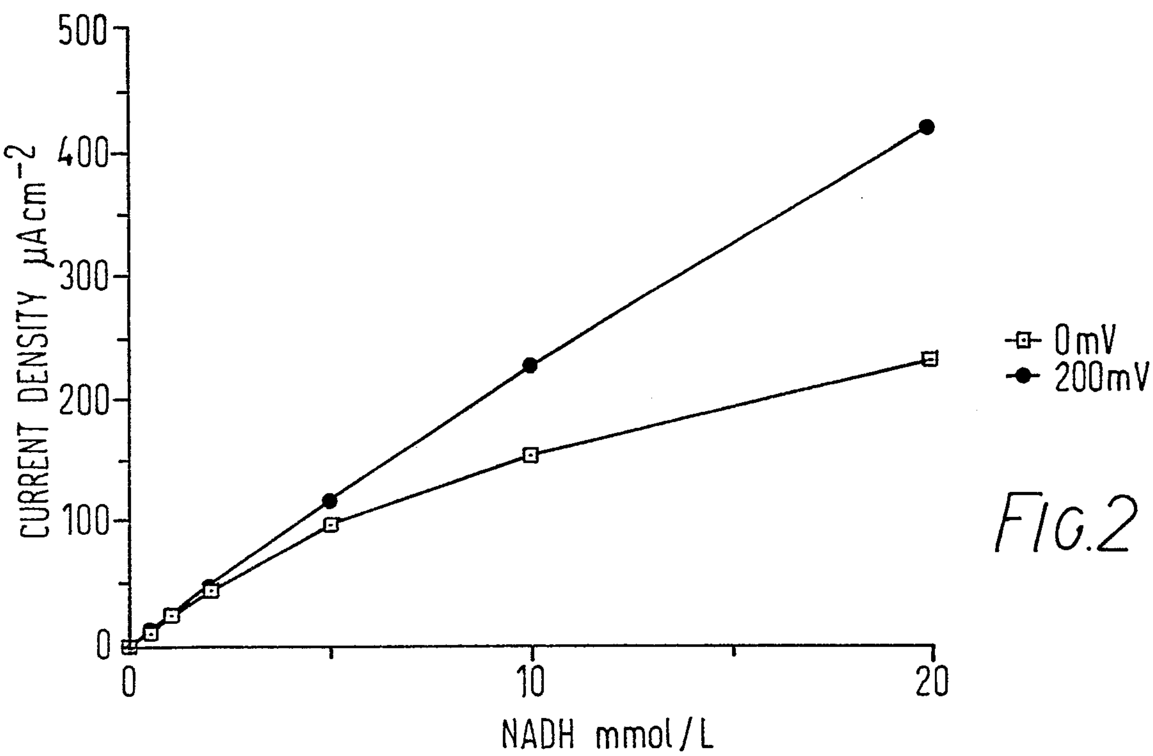
500
400
300
200
100
0
CURRENT DENSITY µA cm⁻²
0
10
20
NADH mmol/L
0mV
200mV

FIG.2

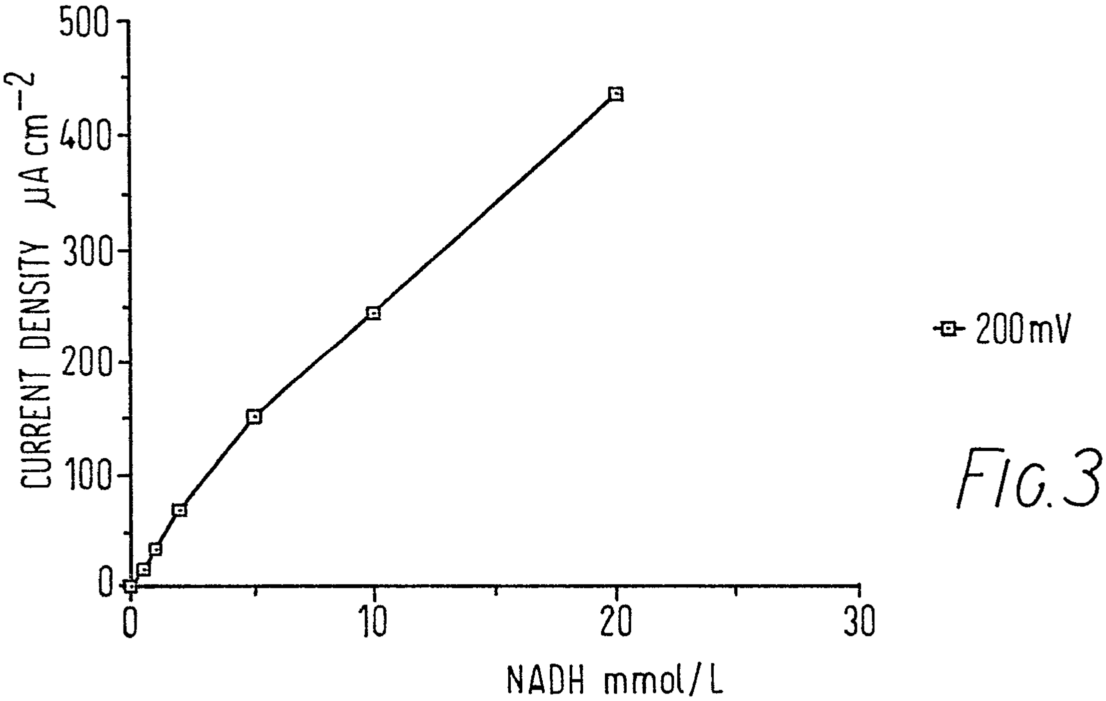

500
400
300
200
100
0
CURRENT DENSITY μA cm⁻²
0
10
20
30
NADH mmol/L
200mV


FIG.3

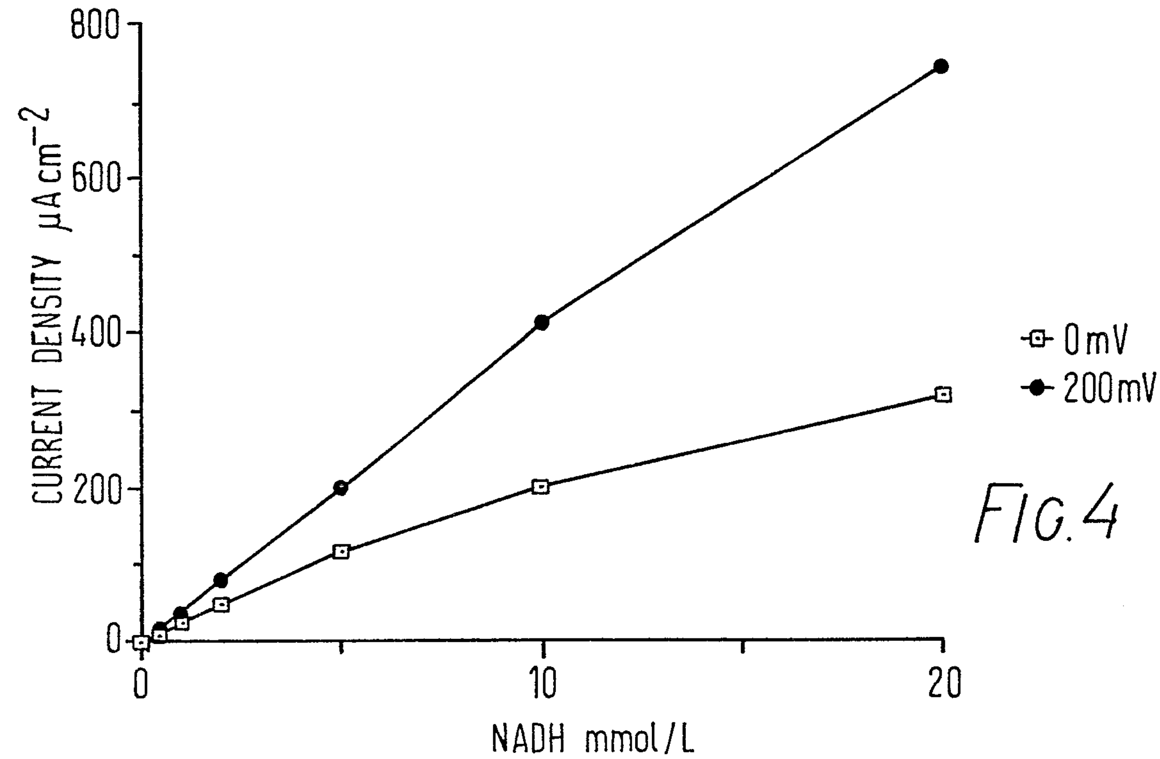

800
600
400
200
0
CURRENT DENSITY μA cm⁻²
0
10
20
NADH mmol/L
0mV
200mV


FIG.4

European Patent Office

# EUROPEAN SEARCH REPORT

Application Number

EP 88 30 3922

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A D | EP-A-0 247 850 (CAMBRIDGE LIFE SCIENCES) * Abstract; page 4, lines 18-41; claims * | 1-16 | C 12 M 1/40<br>G 01 N 33/48 |
| A,D | US-A-4 166 143 (PETROW et al.) * Abstract; claim 1 * | 1-10,14-16 | |
| | | | **TECHNICAL FIELDS SEARCHED (Int. Cl.4)** |
| | | | C 12 M<br>G 01 N |

The present search report has been drawn up for all claims

| Place of search | Date of completion of the search | Examiner |
|---|---|---|
| THE HAGUE | 05-07-1988 | EPAILLARD P.J.H.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document